# EUROPÄISCHE PATENTSCHRIFT

(11) **EP 0 563 953 B1**
(45) Veröffentlichungstag und Bekanntmachung des Hinweises auf die Patenterteilung: **05.06.1996**
(21) Anmeldenummer: 93105410.0
(22) Anmeldetag: 01.04.1993
(51) Int. Cl.: A61N 5/06, A61B 5/00

(54) **Vorrichtung zum Applizieren mit Licht**
Device for applying light
Dispositif d'application de lumière

(30) Priorität: 03.04.1992 DE 9204621 U
(43) Veröffentlichungstag der Anmeldung: 06.10.1993
(73) Patentinhaber: ORALIA DENTALPRODUKTE GmbH, D-78467 Konstanz (DE)
(72) Erfinder: Warnke, Ulrich, Dr., W-6601 Scheidt (DE); Meier, Wolfgang, CH-8272 Ermatingen (CH)
(74) Vertreter: Müller, Hans-Jürgen, Dipl.-Ing.

(56) Entgegenhaltungen:
- EP-A- 0 406 454
- EP-A- 0 462 302
- WO-A-89/00871
- DE-A- 2 846 471
- DE-A- 3 012 150
- US-A- 4 648 892
- F.Frank,G.Hauptmann "Dosiswirkungsgeregeltes Nd:YAG Lasersystem zum Schneiden von Gewebe" in Angewandte Lasermedizin, Hrsg Berlien, Müller, 1989 ecomed Verlagsgesellschaft mbH, Landsberg, München, Zürich, Kapitel II-4.1.1. Seiten 1-4, 4.Erg.Lfg 10/91

## Beschreibung

Die Erfindung bezieht sich auf eine Vorrichtung der im Oberbegriff des Anspruchs 1 genannten Gattung.

Eine solche Vorrichtung ist bereits bekannt (Angewandte Lasermedizin 10/91, Seiten 1-14). Diese vorbekannte Vorrichtung dient zum Schneiden von Geweben mittels energiereicher Strahlen. Dabei werden die Lichtleiterenden an das Gewebe gelegt; sie bilden die Kontaktfläche und begünstigen durch die Kontaminierung den operativen Schneidvorgang. Beim Karbonisieren des "verbrennenden" Gewebes entsteht ein Sekundärlicht, auf das der Sensor anspricht. Diese Vorrichtung läßt sich aber nur bei solchen "Verbrennungsvorgängen" anwenden.

Ferner ist es bekannt (DE-U-9 017 070), bei einer steuerbaren Lichtabgabeeinrichtung zum Aushärten von lichtempfindlichen Dentalwerkstoffen einen Teil des auf Lichtleitern geführten Lichts vom Lichtleiter "auszublenden" und einem Komparator zuzuführen, der eine Steuereinrichtung für die Dosierung der Lichtenergie steuert. Dabei befindet sich im Lichtstrahl ein Spektralfilter. Auch hier ist die Anwendung auf einen Spezialzweck beschränkt.

Darüber hinaus ist es bekannt (DE-A-2 845 471), optische Bestrahlungsgeräte mit unterschiedlichen Filtern zu bestücken, um diese wahlweise als Polymerisations- oder als Koagulaationsgerät anzuwenden.

Bei einem lichtdiagnostischen Untersuchungs und Behandlungsgerät ist es bekannt (US-A-4 648 892), Laserlicht über einen Lichtleiter und einen Applikator an den zu diagnostizierenden Venen- oder Arterienbereich zu leiten. Von dort reflektiertes Licht wird in einer Abbildungseinrichtung zum Feststellen von z.B. Anomalien ausgewertet. Sofern ein hochenergetischer Zusatzlaser verwendet wird, kann auch erkranktes Gewebe verbrannt werden. Andere Anwendungen sind nicht vorgesehen.

Darüber hinaus ist es bekannt (US-A-4 718 417, DE-C-2 829 516 und DE-A-3 319 203), Primärlicht zu einer Photokoagulation in Gewebe oder dergleichen zu verwenden. Das Licht wird mit anderer Wellenlänge vom Gegenstand emittiert. Dem Koagulationsgrad entsprechende thermische Strahlung wird als Sekundärlicht vom Sensor ermittelt, der gut gegen Primärlicht abgeschirmt ist.

Ferner ist es bekannt, polarisiertes Licht, insbesondere LASER-Licht, auf Zellen und Gewebe zu applizieren, wodurch beispielsweise Wachstumsfaktoren stimuliert werden. Auch langwellige Wärmestrahlen, kurzwellige UV-Strahlen und nicht kohärente linear polarisierte Lichtstrahlen werden zum Applizieren von organischen Substanzen, beispielsweise lebendem Gewebe, verwendet, um beispielsweise die Wundheilung zu fördern oder Muskelverspannungen zu lösen oder oberflächliche Bräunungseffekte zu erzielen. Die Dosierung solcher Laserstrahlen, UV-Strahlen und dergl. ist jedoch nicht einfach zu bewerkstelligen, wenn sich die zu applizierende Lichtenergiemenge nur in einem beschränkten Toleranzbereich befinden darf, um unerwünschte Beschädigungen des applizierten Gegenstandes, beispielsweise Beschädigung von Hautgeweben, zu vermeiden.

Der Erfindung liegt die Aufgabe zugrunde, eine Vorrichtung der eingangs genannten Gattung mit einfachen Mitteln dahingehend zu verbessern, daß mit ihrer Hilfe höchst unterschiedliche Arbeiten möglich sind. Eine genaue Dosierung soll möglichst automatisch erfolgen können. Darüber hinaus ist erwünscht, den Energiebedarf insgesamt gering zu halten.

Die Erfindung ist im Anspruch 1 gekennzeichnet und weitere Ausbildungen derselben sind in Unteransprüchen beansprucht und werden in der folgenden Beschreibung auch anhand der Zeichnung näher erläutert.

Bei der Erfindung wird ein insbesondere aus Glas- oder Kunststoffasern bestehender Lichtleiter dazu verwendet, das Licht auf möglichst der gesamten Strecke zwischen der Lichtquelle und dem Applikator zu leiten und "Luftübergänge" weitgehend zu vermeiden. Dieser weist eine Kontaktfläche auf, welche einerseits den Lichtübergang zum zu bestrahlenden Gegenstand gewährleistet, andererseits in Verbindung mit der an die Kontaktfläche angelegten Oberfläche des Gegenstands aber auch eine "Reflexionsfläche" bildet, welche nicht auf den Gegenstand übertragenes Primärlicht bzw. Applikationslicht durch Reflexion, Steuung, Beugung oder dergl. wieder aufnimmt und über insbesondere den gleichen Lichtleiter, der schon zum Hinführen des Lichts zur Kontaktfläche diente, zu einem Sensor zu leiten, welcher dieses zurückgeleitete Primärlicht erfaßt. Dies ist unterschiedlich zu dem in DE-U-90 17 070 gemachten Vorschlag, einen Teil des vom Sensor zum Lichtleiterende geleiteten Lichts auszublenden und zum Steuern der Lichtquelle zu verwenden. Das Ausgangssignal des Sensors gelangt bei der Erfindung zu einem Komparator, welcher als Vergleichseinrichtung dieses vom Sensor erfaßte Licht bzw. einen davon abgeleiteten Signalwert mit einem Vergleichswert vergleicht. Das Vergleichsergebnis dieses Vergleichs im Komparator dient als Steuergröße für eine Steuereinrichtung, welche die Dosiereinrichtung zum richtigen Dosieren der von der Lichtquelle abzugebenden Lichtenergiemenge steuert. Die Steuereinrichtung kann zusätzlich auch eine Anzeigeeinrichtung steuern, falls zusätzliche Anzeigeaufgaben gelöst werden sollen.

Damit möglichst wenig Energie verlorengeht, empfiehlt sich die Verwendung einer Kaltlichtquelle, insbesondere einer Halogenlampe, der zusätzlich ein Wärmefilter vorgesetzt sein kann. Auch ist es empfehlenswert, die Kaltlichtquelle mit Gleichspannung zu speisen, d. h. zwischen die Lichtquelle und eine Wechselstromquelle einen Gleichspannungswandler einzuschalten.

Nach einer besonderen Ausbildung der Erfindung wird das zu applizierende Licht aus einem begrenzten Farbspektrum ausgewählt. Kommt es nämlich darauf an, nur eine sehr dünne Schicht des Gegenstandes, insbesondere deren Oberfläche, zu bestrahlen, empfiehlt sich die Verwendung eines UV- und Blaufilters mit Lichtenergie insb. zwischen etwa 320 und 450 nm Wellenlänge im Strahlengang. Ist eine Melanin-Detektion des Absorptionswertes brauner Haut vorgesehen, empfiehlt sich ein Grünfilter. Ein gelbes Farbfilter empfiehlt sich für die Bestrahlung tieferer Gewebeschichten und Rotfilter, die bis in den IR-A-Bereich um etwa 900 nm reichen, werden vor allem für die Behandlung tieferer Schichten im Gegenstand, beispielsweise tieferer Gewebeschichten bis zu etwa 5 cm von der Gewebeoberfläche aus, angewendet.

Die Lichtenergiemenge richtet sich also nicht nur nach der Zeit, Dauer und Intensität des Lichte, sondern auch nach dem Frequenzbereich, aus dem es stammt, d.h. der Lichtfarbe. Im Falle der Ausnutzung der Heilwirkung von polarisiertem Licht empfiehlt sich eine Energiemenge im Bereich von 100 mJ/cm² bestrahlter Fläche. Sofern tiefere Gewebeschichten bestrahlt werden, kann eine auch erheblich höhere Lichtmenge angewendet werden.

Das polarisierte Licht ist vorzugsweise linear und zirkular polarisiert. Absorbiert das bestrahlte Gewebe die Photonen nicht, so erfolgt aufgrund unterschiedlicher Brechungsindizes und der dadurch bedingten unterschiedlichen Lichtgeschwindigkeit eine Drehung der Polarisationsebene des Lichtes. Für Wellenlängen, bei denen der betreffende Gegenstand absorbiert, wird der Lichtstrahl eliptisch polarisiert und die Hauptachse der Ellipse gedreht. Anisotrope Gewebe machen aus linear polarisiertem Licht im Durchlässigkeitsbereich eine eliptische Polarisation. Ein Unterfall ist das eliptisch und zirkular polarisierte Licht.

Es empfiehlt sich bei Anwendung linear polarisierten Lichts die Anwendung eines Neigungswinkels der Kontaktfläche, d. h. eines Eintrittswinkels des Lichtes vom Lichtleiter in den Gegenstand, beispielsweise Hautgewebe; sofern der Neigungswinkel zwischen etwa 50 und 60 Grad aufweist, wird eine besonders große Verlustarmut an der Kontaktfläche erzielt.

Für viele Anwendungsfälle empfiehlt es sich, wenn der Applikator mit seiner mit dem Gegenstand in Kontakt tretenden Kontaktfläche vom Lichtleiter abtrennbar ist. Zu diesem Zweck ist es zweckmäßig, den Applikator mittels einer Kupplung mit einem Handgriff zu verbinden, so daß der Applikator nach dem Entkuppeln abgenommen und beispielsweise sterilisiert werden kann.

Das Polarisationsfilter ist zweckmäßigerweise zwischen der Kontaktfläche und dem Lichtleiterende angeordnet, was nach einer besonderen Ausbildung der Erfindung in Form einer Kappe geschieht, die zwischen das Lichtleiterende und den Applikator so eingefügt wird, daß der von der Stirnseite des Lichtleiterendes ausgehende Lichtstrahl durch das Polarisationsfilter auf das Kontaktelement fällt, welches die Kontaktfläche an der Außenseite des Applikators bildet. Für manche Anwendungsfälle kann das Polarisationsfilter auch abgenommen sein.

Bei anderen Anwendungsfällen, bei denen es auf eine möglichst punktförmige Applikation des Lichtes ankommt, empfiehlt es sich, im Bereich zwischen dem Lichtleiterende und der Kontaktfläche eine Fokussiereinrichtung anzuordnen. Hierdurch können beispielsweise Akupunktur-Punktstimmulationen erreicht werden, da eine hohe Photonendichte von mehr als beispielsweise 800 mW/cm² in allen Farbtemperaturen auf der Hautoberfläche erzielbar ist.

Ein bevorzugtes Ausführungsbeispiel der Erfindung wird nun anhand der Zeichnung erläutert. Darin zeigen:
- Figur 1: eine schematische Darstellung -teilweise im schematischen Schnitt- einer erfindungsgemäßen Vorrichtung und
- Figur 2: einen vergrößerten Teilschnitt des Applikators im Bereich des Lichtleiterendes.

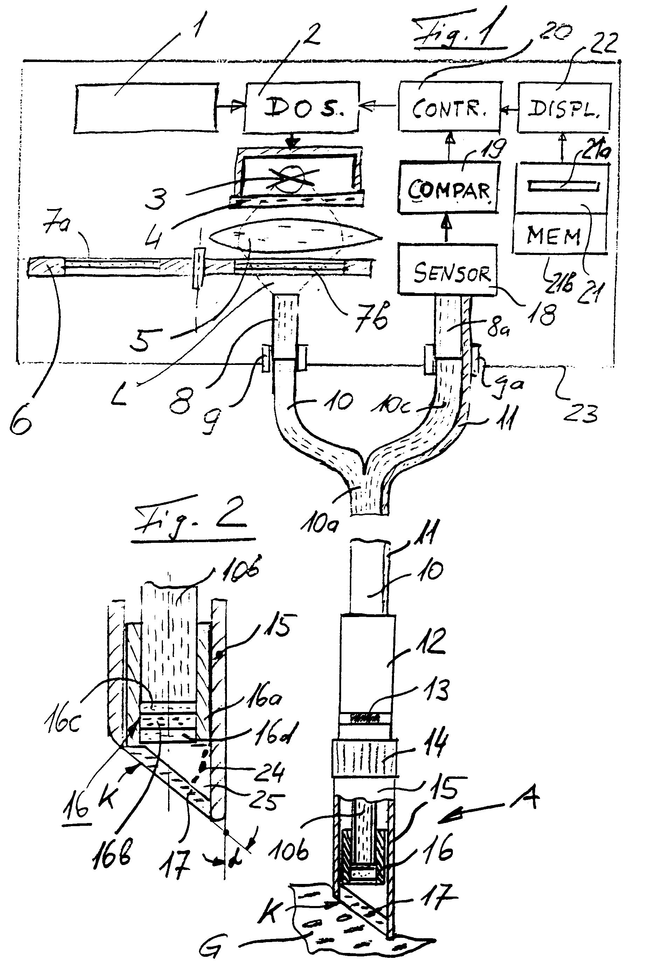

Bei der erfindungsgemäßen Vorrichtung wird als Lichtquelle 3 eine Halogenlampe verwendet. Die Steuerung, darunter das Ein- und Ausschalten der Lichtquelle 3, erfolgt über die Dosiereinrichtung 2, welcher eine Spannungsquelle 1 vorgeschaltet ist, welche die Netz-Wechselspannung in Gleichspannung umformt. Die Dosiereinrichtung 2 wird von einer Steuereinrichtung CONTR 20 gesteuert. Das von der Lichtquelle 3 ausgesandte weiße Licht durchtritt ein Wärmefilter 4, welches Wärmestrahlen ausfiltert. Danach gelangt das Licht L durch eine Sammellinse 5 und ein Farbfilter 7b, beispielsweise ein Blaufilter, das mit anderen Farbfiltern, beispielsweise dem Gelbfilter 7a, auf einer als drehbarer Karusselplatte dienenden Mehrfiltereinrichtung 6 angeordnet ist, so daß wahlweise eines von beispielsweise vier verschiedenen Farbfiltern in den Strahlengang des Lichts L eingeschaltet werden kann. Der Antrieb der Mehrfilteranordnung 6 kann manuell, aber auch automatisch durch eine Steuereinrichtung erfolgen. Das Licht tritt als Applikationslicht L in einen Lichtkoppler 8 in Form eines Lichtleiters ein, welcher das Licht zu einer Anschlußbuchse 9 des von dem Gehäuse 23 umgebenden Geräts leitet. Von dort tritt das Licht über die Stirnseite des Lichtkopplers 8 und die Stirnseite des Lichtleiters 10, insb. eines Glasfaserkabels, in diesen ein. Das Licht L wird dann bis zu dem anderen Lichtleiterende 10b geleitet und tritt dort über das Polarisationsfilter 16, welches eines insb. lineare Polarisierung vornimmt, in das Kontaktelement 17 ein, um über die Kontaktfläche K in den Gegenstand G, beispielsweise organisches Gewebe, jedenfalls an der Grenzfläche einzudringen.

Das Polarisationsfilter 16 besteht hier aus einem dünnen Polarisationsfilterblättchen aus Glas- oder Kunststoffolie, welche beidseitig mit einem Glas- oder Kunststoffblättchen abgedeckt ist. Diese drei Segmente werden von einer Hülse 16a gehalten, die als Kappe auf das Lichtleiterende 10b dann aufsteckbar ist, wenn der Applikator K mit seiner metallischen Hülse 15 und dem aus insb. Glas bestehenden Kontaktelement 17 abgezogen ist.

Weitere Anwendungsfälle sind beispielsweise das durch Licht polymerisierende Aushärten von Zahnfüllmaterial, wie dem unter dem Warenzeichen ESTILUX bekannten lichthärtenden Einkomponenten-Composite mit glaskeramischem Füllstoff und/oder hochdispersem Siliziumdioxyd. In diesem Fall kann auf das Polarisationsfilter verzichtet werden.

Die nicht auf den Gegenstand übergegangene Lichtmenge, also ein Teil des Applikationslichts L, gelangt durch Reflexion, Streuung oder Beugung oder dergleichen insb. über den gleichen Lichtleiter 10 wieder zurück in das Ausgangsgerät mit dem Gehäuse 23. Zu diesem Zweck empfiehlt es sich, eine Abzweigung 10a im Lichtleiter vorzusehen, so daß ein Lichtleiterteil 10c zu einer anderen Buchse 9a führt, die über einen anderen Lichtkoppler 8a mit dem Sensor 18 bzw. der dort angeordneten Photodiode verbunden ist. Der vom Sensor ermittelte nicht auf den Gegenstand G übertragene und auch nicht anderweitig verlorengegangene Anteil des Primär- bzw. Applikationslicht führt zu einem Ausgangssignal, welches dem Komparator COMPAR 19 zugeleitet wird. Hier erfolgt nun ein Vergleich mit einem Vergleichswert, beispielsweise einem IST-Wert, um in Abhängigkeit vom Unterschied zwischen diesen beiden Werten die Steuereinrichtung CONTR 20 zu beeinflussen, die ihrerseits auf die Dosiereinrichtung 2 einwirkt, um die richtige Dosierung vorzunehmen.

Für viele Anwendungsfälle empfiehlt sich die Verwendung einer Anzeigeeinrichtung DISPL 22 beispielsweise dann, wenn Messungen mittels Lichtstrahlen am Gegenstand G über die Kontaktfläche K vorgenommen werden sollen.

Die Anzeigeeinrichtung 22 kann jedoch auch zur Anzeige verschiedener Programme dienen, welche beispielsweise durch Einstecken von Datenträgern, wie Disketten oder Memory-Karten, in die Datenträger-Aufnahmeeinrichtung 21a ausgewählt werden. Die Programmauswahleinrichtung 21 kann darüber hinaus einen Datenspeicher MEM 21b aufweisen, in dem bestimmte Programme eingespeichert sind, die dann durch auf den Datenträgern befindliche Befehle abgerufen und dem Komparator 19, der Anzeigeeinrichtung 22, dem Sensor 18, der Steuereinrichtung 20 und/oder der Mehrfiltereinrichtung 66 bzw. der Antrieb zugeführt zu werden.

Darüber hinaus kann eine Programmauswahl und/oder Einschalten bzw. Ausschalten der Dosiereinrichtung 2 auch mittels eines Schalters 13 bzw. Tasters erfolgen, welcher im Handgriff 12 angeordnet ist und über eine insbesondere elektrische Leitung 11 mit der entsprechenden Einrichtung, beispielsweise dem Sensor 18 oder (nicht dargestellt) der Dosiereinrichtung 2, in Verbindung steht. Mit dem Schalter 13 können daher in unmittelbarer Nähe des Applikators A sowohl Programmwahl als auch Programmablauf von der Bedienungsperson gesteuert werden. Dabei besteht auch die Möglichkeit, eine Anzeigeeinrichtung im Handgriff selbst unterzubringen.

Das Anwendungsgebiet der erfindungsgemäßen Vorrichtung ist außerordentlich vielseitig. So sind analytische Untersuchungen an und/oder in Gegenständen möglich. Darüber hinaus kann die Vorrichtung zur heilenden Behandlung von Geweben Anwendung finden. Auch technische Anwendungen wie das oben schon erwähnte Aushärten von lichthärtendem Material sind möglich, ebenso wie die Anwendung als Detektor, um beispielsweise pulpäre Irritationen im Zahnbereich zu ermitteln; im letztgenannten Fall wird zweckmäßigerweise ein Gelbfilter angewendet und die Kontaktfläche K auf die Kaufläche des Backenzahn oder die Frontfläche eines Schneidezahns angelegt, wodurch beispielsweise der Pulsschlag der Pulpa und auch kariöse Erscheinungen über Vergleichswerte bzw. Vergleichsprogramme von gesunden Pulpen feststellbar sind. Mit anderen Worten: Das Gerät dient hier auch als Diagnosehilfe.

Ferner sind Depressionsbehandlungen über Augenbestrahlungen ebenso möglich wie kosmetische Anwendungen, beispielsweise auch die Behandlung von Akne.

Der Sensor 18 dient zum Erfassen der vom Gegenstand nicht absorbierten, sondern von dort reflektierten bzw. von dort zurückgesandten Lichtstrahlen. Durch Vergleich der über den Lichtkoppler 8a aufgenommenen Lichtstrahlen und mit in den Lichtkoppler 8 abgesandten Lichtstrahlen kann der Differenzwert ermittelt werden. Die Multiplikation des auf die betreffende Einrichtung normierten Differenzwerts mit der Applikationszeit der Lichtstrahlen ergibt die vom Gegenstand absorbierte Dosis, und zwar auf automatische Weise, was einen erheblichen technischen Fortschritt sicherstellt.

Durch Anwendung von Blaulicht mit evtl. UV-Anteilen werden Zellmembranen durchlässiger, was Wachstumsfaktoren stimuliert. Die Enzyme in den Mitochondrien werden aktiviert zur ATP-Bildung. Die SH-Enzyme werden reduziert. Bei Anwendung von Rotlicht, ggf. ergänzt durch Gelblicht und IR-A-Licht, wird der Kohlenhydrat-, Eiweiß- und Fettstoffwechsel stimuliert und können ebenfalls Enzyme in den Mitochondrien aktiviert werden, was zur Wundheilung bzw. Schmerzreduzierung Anlaß gibt. Die bei der Erfindung anwendbare polarisierte Photonenapplikation synchron zur Energierelevanz ist ein fortschrittliches Hilfsmittel beispielsweise in der Dentalpraxis.

Es empfiehlt sich, den Applikator A über einen biegbaren Lichtleiter 10 mit dem Gehäuse 23 zu verbinden, so daß die Bedienungsperson den Applikator A mit der Kontaktfläche K auch an sonst schwierig zugängige Stellen in beispielsweise Körperhöhlen, wie der Mundhöhle, einführen und gezielt an die gewünschte Behandlungs- bzw. Untersuchungsfläche anlegen kann.

Außerdem kann es zweckmäßig sein, in der Umgebung der Kontaktfläche K bzw. im Raum 25 einen Reflektor 24, z. B. eine reflektierende Beschichtung, poliertes Metall oder eine besonders konstruierte Reflektorstruktur, anzuordnen, um vom Gegenstand G oder der Kontaktfläche K reflektiertes Licht wieder in das Lichtleiterende 10b, d. h. in die Lichtleiterfasern, einzukoppeln, damit das vom Gegenstand G reflektierte Licht möglichst verlustfrei am Sensor 18 eintrifft.

## Patentansprüche

1. Vorrichtung zum Applizieren von Lichtenergie, mit einer Lichtquelle (3), mit einem Lichtleiter (10), von dessen Ende (10b) Applikationslicht auf den zu behandelnden Gegenstand (G) über eine Kontaktfläche (K) an diesem Gegenstand (G) aufbringbar ist, mit einem Sensor (18) zum Erfassen von über den Lichtleiter (10) von der Kontaktfläche (K) zurückkehrendem Licht, mit einem Komparator (19) zum vergleichen dieses erfaßten Lichtes oder eines davon abgeleiteten Signals mit anderen insbesondere gespeicherten Informationen, gegebenenfalls mit einer Anzeigeeinrichtung (22), und mit einer Steuereinrichtung (29) und/oder einer Dosiereinrichtung (2), welche die Lichtquelle (3) bzw. die Dosierung der Lichtenergie in Abhängigkeit vom Ausgangssignal des Komparators (19) steuert,
**dadurch gekennzeichnet,**
daß sich die Kontaktfläche (K) im Abstand vom Lichtleiter (10) befindet und Teil eines Kontaktelements (17) eines vom Lichtleiter (10) abnehmbaren Applikators (A) ist, und daß in den Strahlengang zwischen der Lichtquelle (3) und dem Lichtleiter (10) eine Mehrfiltereinrichtung (6) mit Farbfiltern (7a, 7b) unterschiedlicher Filterbereiche eingeschaltet ist, wobei die Farbfilter (7a, 7b) wahlweise je nach Behandlungsart des an die Kontaktfläche (K) angelegten Gegenstands (G) in den Stahlengang einrückbar sind.

2. Vorrichtung nach Anspruch 1,
**dadurch gekennzeichnet**,
daß ein Polarisationsfilter (16) zum Polarisieren des Applikationslichtes in dessen Strahlengang einschaltbar ist.

3. Vorrichtung nach Anspruch 2,
**dadurch gekennzeichnet**,
daß das innerhalb des Applikators (A) befindliche Lichtleiterende (10b) stirnseitig vom Polarisationsfilter (16) abgedeckt ist.

4. Vorrichtung nach einem der vorhergehenden Ansprüche1,
**dadurch gekennzeichnet**,
daß der Applikator (A) mittels einer Kupplung (14) mit einem Handgriff (12) verbindbar ist, zu dem der Lichtleiter (10) führt, und daß der Handgriff (12) einen Schalter (13) oder Taster aufweist, welcher über einen elektrischen Leiter (11) mit der Steuereinrichtung (20), der Dosiereinrichtung (2), der Anzeigeeinrichtung (22), einem Antrieb für die Mehrfiltereinrichtung (6) und/oder einer Programmauswahleinrichtung (21) in Steuerverbindung steht.

5. Vorrichtung nach Anspruch 4,
**dadurch gekennzeichnet**,
daß der Applikator (A) ein transparentes Kontaktelement (17) aufweist, dessen Kontaktfläche (K) unter einem Neigungswinkel (α) zur Längsachse des Applikators (A) bzw. Handgriffs (12) geneigt ist.

6. Vorrichtung nach einem der vorhergehenden Ansprüche,
**dadurch gekennzeichnet**,
daß in der Nähe des Kontaktelements (K) ein Reflektor (24) zum Reflektieren des vom bestrahlten Gegenstand (G) reflektierten Lichts zurück in den Lichtleiter (10) angeordnet ist.

7. Vorrichtung nach einem der vorhergehenden Ansprüche,
**dadurch gekennzeichnet**,
daß der Applikator (K) mit einer Fokussiereinrichtung versehen ist, welche die Lichtstrahlen auf einen im wesentlichen punktförmigen Bereich der Kontaktfläche (K) fokussiert.

8. Vorrichtung nach einem der vorhergehenden Ansprüche,
**dadurch gekennzeichnet**,
daß als Lichtquelle (3) eine Kaltlichtquelle verwendet ist, der insbesondere ein Wärmefilter (4) vorgesetzt ist.

9. Vorrichtung nach einem der vorhergehenden Ansprüche,
**dadurch gekennzeichnet**,
daß eine Programmauswahleinrichtung (21) eine Datenträger-Aufnahmeeinrichtung (21a) sowie einen Datenspeicher (21b) aufweist, deren Daten zur Steuerung der Dosiereinrichtung (2), Steuereinrichtung (20) und/oder Mehrfilteranordung (6) dienen.

## Claims

1. A device for applying light energy, comprising a light source (3), a fiber-optical light guide (10) from whose end (10b) application light may be applied onto the object to be treated via a contact surface (K) on said object, a sensor (18) for detecting light returning from the contact surface (K) via the fiber-optical light guide (10), a comparator (19) for comparing said detected light or a signal derived therefrom with other information, more particularly stored information, an optional display means (22) and a control means (29) and/or a dosing means (2) for controlling the light source (3) or, respectively, the dosing of the light energy in response to the output signal from the comparator (19),
**characterised in**
that the contact surface (K) is spaced apart from the fiber-optical light guide (10) and forms part of a contact member (17) of an applicator (A) detachable from the fiber-optical light guide (10) and that a multi-filter means (6) comprising colour filters (7a, 7b) of differing filter ranges is inserted in the beam path between the light source (3) and the fiber-optical light guide (10), the colour filters (7a, 7b) being optionally movable into the beam path according to the respective type of treatment of the object (G) bearing against the contact surface (K).

2. A device according to claim 1,
**characterised in**
that a polarization filter (16) for polarizing the application light may be inserted in the beam path thereof.

3. A device according to claim 2,
**characterised in**
that the light guide end (10b) situated within the applicator (A) is covered, on the front side thereof, by a polarization filter (16).

4. A device according to any of the preceding claims,
**characterised in**
that the applicator (A) may be connected with a handle (12), to which the light guide (10) leads, via a coupling (14) and that the handle (12) comprises a switch (13) or a button key being in control connection, via an electrical conductor (11), with the control means (20), the dosing means (2), the display means (22), a drive for the multi-filter means (6) and/or a program select means (21).

5. A device according to claim 4,
**characterised in**
that the applicator (A) comprises a transparent contact member (17) whose contact surface (K) is inclined at an inclination angle (α) with respect to the longitudinal axis of the applicator (A) or the handle (12), respectively.

6. A device acording to any preceding claims,
**characterised in**
that a reflector (24) for reflecting the light reflected from the irradiated object (G) back into the fiber-optical light guide (10) is disposed in the vicinity of the contact member (K).

7. A device according to any of the preceding claims,
**characterised in**
that the applicator (K) is provided with a focussing means for focussing the light beams onto a substantially punctual region of the contact member (K).

8. A device according to any of the preceding claims,
**characterised in**
that a could light source is used as the light source (3), an IR filter (4) being disposed in front thereof, in particular.

9. A device according to any of the preceding claims,
**characterised in**
that a program select means (21) comprises a data carrier receiving means (21a) as well as a data memory (21)b), the data thereof serving for controlling the dosing means (2), the control means (20) and/or the multi-filter means (6).

## Revendications

1. Dispositif d'application d'énergie lumineuse, comportant une source lumineuse (3), un guide de lumière (10), depuis l'extrémité (10b) duquel la lumière à appliquer sur l'objet (G) à traiter est susceptible d'être amenée par l'intermédiaire d'une surface de contact (K) située sur cet objet (G), un détecteur (18) pour détecter la lumière revenant de la surface de contact (K) par l'intermédiaire du guide de lumière (10), un comparateur (19) pour comparer cette lumière détectée ou un signal dérivé de celle-ci avec d'autres informations, en particulier des informations mémorisées, le cas échéant un dispositif de visualisation (22) et un dispositif de commande (29) et/ou un dispositif de dosage (2) qui commande la source lumineuse (3) ou le dosage de l'énergie lumineuse, respectivement, en fonction du signal de sortie du comparateur (19),
**caractérisé en ce que**
la surface de contact (K) se trouve à distance du guide de lumière (10) et fait partie d'un élément de contact (17) d'un applicateur (A) amovible du guide de lumière (10), et en ce qu'un dispositif à filtres multiples (6) avec filtres chromatiques (7a, 7b) de différentes gammes de filtrage est intercalé dans la marche des rayons entre la source de lumière (3) et le guide de lumière (10), les filtres chromatiques (7a, 7b) étant susceptibles d'être introduits au choix dans la marche des rayons, selon le type de traitement de l'objet (G) en appui sur la surface de contact (K).

2. Dispositif selon la revendication 1,
**caractérisé en ce**
qu'un filtre de polarisation (16) pour polariser la lumière d'application est susceptible d'être intercalé dans sa marche des rayons.

3. Dispositif selon la revendication 2,
**caractérisé en ce que**
l'extrémité (10b) du guide de lumière, qui se trouve à l'intérieur de l'applicateur (A), est couverte sur la face frontale par le filtre de polarisation (16).

4. Dispositif selon l'une quelconque des revendications précédentes,
**caractérisé en ce que**
l'applicateur (A) peut être relié par l'intermédiaire d'un accouplement (14) à une poignée (12) à laquelle mène le guide de lumière (10) et en ce que la poignée (12) présente un commutateur (13) ou une touche qui est en liaison de commande par l'intermédiaire d'un conducteur électrique (11) avec le dispositif de commande (20), le dispositif de dosage (2), le dispositif de visualisation (22), un entraîne- ment pour le dispositif à filtres multiples (6) et/ou un dispositif de sélection de programmes (21).

5. Dispositif selon la revendication 4,
**caractérisé en ce que**
l'applicateur (A) présente un élément de contact (17) transparent dont la surface de contact (K) est inclinée sous un angle d'inclinaison (a) par rapport à l'axe longitudinal de l'applicateur (A) ou de la poignée (12), respectivement.

6. Dispositif selon l'une quelconque des revendications précédentes,
**caractérisé en ce**
qu'un réflecteur (24) est agencé à proximité de l'élément de contact (K) pour réfléchir en retour dans le guide de lumière (10) la lumière réfléchie par l'objet (G) irradié.

7. Dispositif selon l'une quelconque des revendications précédentes,
**caractérisé en ce que**
l'applicateur (K) est pourvu d'un dispositif de focalisation qui focalise les rayons lumineux sur une zone sensiblement ponctuelle de la surface de contact (K).

8. Dispositif selon l'une quelconque des revendications précédentes,
**caractérisé en ce**
qu'on utilise comme source lumineuse (3) une source lumineuse froide devant laquelle est placé un écran à l'infrarouge (4).

9. Dispositif selon l'une quelconque des revendications précédentes,
**caractérisé en ce**
qu'un dispositif de sélection de programmes (21) présente un dispositif de réception d'un support de données (21a) ainsi qu'une mémoire de données (21b) dont les données servent à la commande du dispositif de dosage (2), du dispositif de commande (20) et/ou du dispositif à filtres multiples (6).
